# EUROPÄISCHE PATENTSCHRIFT

(11) **EP 3 870 228 B1**
(45) Veröffentlichungstag und Bekanntmachung des Hinweises auf die Patenterteilung: **03.01.2024**
(21) Anmeldenummer: 18811138.9
(22) Anmeldetag: 22.10.2018
(51) Int. Cl.: A61K 31/4045, A61K 9/50, A61K 47/14

(54) **HOT-MELT-COATING-VERFAHREN**
HOT-MELT COATING PROCESS
PROCÉDÉ DE REVÊTEMENT THERMOFUSIBLE

(43) Veröffentlichungstag der Anmeldung: 01.09.2021
(73) Patentinhaber: IOI Oleo GmbH, 20459 Hamburg (DE)
(72) Erfinder: LOCHMANN, Dirk, 58730 Fröndenberg (DE); REYER, Sebastian, 45149 Essen (DE); STEHR, Michael, 45881 Gelsenkirchen (DE); ZIMMER, Andreas, 8010 Graz (AT); SALAR BEHZADI, Sharareh, 1080 Wien (AT)
(74) Vertreter: Kayser, Christoph
(86) Internationale Anmeldenummer: PCT/DE2018/000302
(87) Internationale Veröffentlichungsnummer: WO 2020/083411

(56) Entgegenhaltungen:
- EP-A1- 2 198 859
- EP-A2- 0 451 461
- WO-A1-2011/110926
- US-A- 5 891 476
- BECKER KARIN ET AL: "Solvent-Free Melting Techniques for the Preparation of Lipid-Based Solid Oral Formulations", PHARMACEUTICAL RESEARCH, SPRINGER NEW YORK LLC, US, Bd. 32, Nr. 5, 19. März 2015 (2015-03-19), Seiten 1519-1545, XP035444577, ISSN: 0724-8741, DOI: 10.1007/S11095-015-1661-Y [gefunden am 2015-03-19]
- LOPES DIOGO GOMES ET AL: "Designing optimal formulations for hot-melt coating", INTERNATIONAL JOURNAL OF PHARMACEUTICS, Bd. 533, Nr. 2, 24. August 2017 (2017-08-24), Seiten 357-363, XP085270336, ISSN: 0378-5173, DOI: 10.1016/J.IJPHARM.2017.08.086

## Beschreibung

Vorgestellt werden Hot-Melt-Coating-Verfahren, kurz HMC-Verfahren, mit einem Coatingmaterial, das auch bei der Herstellung von Arzneimitteln zum Einsatz kommen kann.

HMC-Verfahren bieten gegenüber anderen Beschichtungsverfahren Vorteile. Bei einem HMC-Verfahren wird disperses Material, beispielsweise Feststoffpartikeln in einem Gas oder Gasgemisch als Dispergens, mit dem Coatingmaterial besprüht, wobei das disperse Material üblicherweise als Wirbelschicht bereitgestellt wird. Disperses Material bezeichnet dabei solches Material, das von dem umgebenden Medium durch eine Phasengrenzfläche getrennt ist. Im Gegensatz zu anderen Verfahren ist die Viskosität von geschmolzenen HMC-Coatings für den Verfahrensschritt des Versprühens niedrig genug, so dass sich der Einsatz von Lösungsmitteln, wie beispielsweise Wasser, zur Viskositätserniedrigung erübrigt. Damit können auch energie- und zeitaufwändige Trocknungsschritte entfallen. Auch die Gefahr, dass sich das disperse Material unerwünschterweise während des Beschichtungsvorgangs in dem Coatingmaterial teilweise löst, ist bei lösungsmittelfreien HMC-Coatings deutlich herabgesetzt. In der Lebensmittelindustrie wird das HMC-Verfahren bereits häufig eingesetzt. Für die Herstellung von Arzneimitteln ist die Ummantelung von Granulaten, Kristallen oder allgemein Partikeln, noch allgemeiner, auch beispielsweise Flüssigkeitströpfchen einschließend, von dispersem Material, das einen oder mehrere pharmazeutische Wirkstoffe aufweist, häufig geboten, um beispielsweise unangenehmen Geschmack zu maskieren, den Wirkstoff vor Umwelteinflüssen, wie beispielsweise Feuchtigkeit oder UV-Strahlung, zu schützen oder die Geschwindigkeit der Wirkstofffreisetzung zu beeinflussen. Um auch in der Arzneimittelherstellung HMC-Verfahren einsetzen zu können, müssen die HMC-Coatings besondere Anforderungen erfüllen. Sie müssen insbesondere über einen längeren Zeitraum stabil bleiben und dürfen sich in ihren physikochemischen Eigenschaften möglichst über Jahre nur innerhalb engster Grenzen verändern, um eine gleich bleibende Freisetzungskinetik des pharmazeutischen Wirkstoffs oder einen ausreichenden Schutz bei längerer Lagerung gewährleisten zu können. Als Hot-Melt-Coating oder HMC-Verfahren wird hier jedes Verfahren bezeichnet, bei dem disperses Material mit lösungsmittelfreiem Coatingmaterial ummantelt wird, indem das Coatingmaterial als Schmelze aufgesprüht wird, wobei die Größe der Einzelteile des dispersen Materials grundsätzlich frei wählbar ist. Das dabei verwendete Coatingmaterial wird nachfolgend auch als HMC-Coating oder HMC-Coatingmaterial bezeichnet.

Inzwischen sind verschiedene HMC-Coatings bekannt, die zumeist für bestimmte pharmazeutische Wirkstoffe entworfen wurden. WO 2014/167124 A1 benennt explizit das Problem, dass Triglyceride, wie Glyceroltripalmitat oder Glyceroltristearat, Polymorphismen aufweisen, also jeweils sowohl in einer kristallinen, instabilen α-Modifikation als auch in einer metastabilen β'-Modifikation oder einer stabilen β-Modifikation vorliegen und von einer in die andere Modifikation übergehen können. Die Modifikationen unterscheiden sich dabei insbesondere durch die Dicke lamellar gepackter, kristalliner Untereinheiten, die auch als subzelluläre Einheiten bezeichnet werden. Für die α-Modifikation von Glyceroltristearat konnte unter bestimmten Bedingungen beispielsweise eine eine Schichtung von im Mittel je 6 lamellaren Strukturen pro subzellulärerer Einheit bestimmt werden, nach vollständiger Überführung in die β-Modifikation dann eine Schichtung von im Mittel 10,5 lamellaren Strukturen pro subzellulärer Einheit und eine Zunahme der Kristalldicke um etwa 67 %. Dass dabei die rechnerisch zu erwartende Zunahme um 75 % nicht erreicht wird, ist vermutlich dem Umstand geschuldet, dass die Einzellamellen der β-Modifikation aufgrund einer gegenüber der α-Modifikation auftretenden Schrägstellung eine dichtere lamellare Packung aufweisen (siehe D. G. Lopes, K. Becker, M. Stehr, D. Lochmann et al. in Journal of Pharmaceutical Sciences 104: 4257-4265, 2015). Da die α-Modifikation bei den möglichst niedrig gehaltenen Temperaturen während des Besprühens im HMC-Verfahren eine schnellere Entstehungskinetik als die β'- und die β-Modifikation aufweist, liegt nach mittels HMC-Verfahren erfolgter Ummantelung wirkstoffhaltigen dispersen Materials die α-Modifikation vor, die sich jedoch unter als "blooming" bekannter Volumenzunahme der Ummantelung, die auch mit makroskopischen Aufbrechungen einher geht, während der Lagerung unerwünschterweise in die stabilere β-Modifikation umlagert. Die Lösung des erörterten Problems erfolgt gemäß WO 2014/167124 A1 durch Zusatz eines Polysorbates, also eines nicht-ionischen Tensids, wobei der Polysorbatanteil 10 - 30 % des Coatingmaterials beträgt. Die Mischung aus Triglycerid und Polysorbat scheint zwar das Problem des "bloomings" nicht mehr aufzuweisen, kann sich jedoch langfristig entmischen, so dass eine Veränderung der Freisetzungskinetik, die bei pharmazeutischen Formulierungen grundsätzlich stabil sein sollte, nicht ausgeschlossen werden kann.

US 5,891,476 offenbart eine Zusammensetzung mit Carnauba-Wachs, die keine Polymorphie aufweisen soll. Nachteilig sind jedoch die hohen Schmelztemperaturen für Carnauba-Wachs, die zwischen 82°C und 86°C liegen. Für thermolabile Wirkstoffe sind die offenbarten Zusammensetzungen daher ungeeignet, da die Temperaturen im HMC-Verfahren zum Teil 100°C oder mehr betragen müssten mit dem zusätzlichen Nachteil, dass sich das bei Temperaturabnahme sehr schnell verfestigende Wachs in den Leitungen und Düsen der HMC-Vorrichtung leicht festsetzt und dabei Verstopfungen in der Vorrichtung verursacht.

US 2010/0092569 A1 offenbart die Einbettung eines Silikatpulveradsorbats eines Wirkstoffes in eine geschmolzene Lipidmatrix mit nachfolgendem Versprühen und Kühlen zur Herstellung ummantelter Partikel, wobei die Lipidmatrix aus Triglyceriden von gesättigten geradzahligen Fettsäuren mit je 16 bis 22 Kohlenstoffatomen und 3% eines Emulgators besteht, der die Homogenität der Dispersion des Wirkstoffs in der Lipidmatrix gewährleisten soll. Auch diese Herstellungsweise erfordert Temperaturen oberhalb 70°C und ist somit für temperatursensitive Wirkstoffe ungeeignet. Das offenbarte Verfahren dient der Herstellung eines Tiernahrungsmittels; die für Humanarzneimittel kritische Freisetzungskinetik des Wirkstoffs wird in der benannten Druckschrift nicht adressiert.

Vor diesem Hintergrund stellt sich die Aufgabe, ein Coatingmaterial für ein HMC-Verfahren und mit einem Coating ummanteltes disperses Material bereit zu stellen, das die zuvor erörterten Nachteile des Stands der Technik nicht aufweist, ebenso wie ein HMC-Verfahren, das auch für die Ummantelung dispersen Materials geeignet ist, das einen oder mehrere pharmazeutische Wirkstoffe aufweist, für das eine Zusammensetzung eines Coatingmaterials verwendet wird, das wünschenswerterweise weder vor noch nach der Ummantelung der verwendeten Zusammensetzung Entmischungen aufweist, keine polymorphiebedingten Modifikationsänderungen zeigt, die mit einer Volumenänderung der Ummantelung einhergehen, sowie zudem erforderlichenfalls eine stabile Freisetzungskinetik gewährleistet und dabei, falls nötig, auch zusammen mit dispersem Material, das thermolabile pharmazeutische Wirkstoffe aufweist, verarbeitet werden kann. Unter einem pharmazeutischen Wirkstoff wird hier ein Stoff verstanden, der als pharmakologisch aktiver Bestandteil eines Arzneimittels verwendbar ist. Stoffe sind hier chemische Elemente und chemische Verbindungen sowie deren natürlich vorkommende Gemische und Lösungen, Pflanzen, Pflanzenteile, Pflanzenbestandteile, Algen, Pilze und Flechten in bearbeitetem oder unbearbeitetem Zustand, Tierkörper, auch lebender Tiere, sowie Körperteile, Körperbestandteile und Stoffwechselprodukte von Mensch oder Tier in bearbeitetem oder unbearbeitetem Zustand, Mikroorganismen einschließlich Viren sowie deren Bestandteile oder Stoffwechselprodukte. Arzneimittel sind hier Stoffe oder Zubereitungen aus Stoffen, die zur Anwendung im oder am menschlichen oder tierischen Körper bestimmt sind und als Mittel mit Eigenschaften zur Heilung oder Linderung oder zur Verhütung menschlicher oder tierischer Krankheiten oder krankhafter Beschwerden bestimmt sind oder die im oder am menschlichen oder tierischen Körper angewendet oder einem Menschen oder einem Tier verabreicht werden können, um entweder die physiologischen Funktionen durch eine phamakologische, immunologische oder metabolische Wirkung wiederherzustellen, zu korrigieren oder zu beeinflussen oder eine medizinische Diagnose zu erstellen. Ebenfalls als Arzneimittel gelten hier Gegenstände, die ein Arzneimittel im obigen Sinne enthalten oder auf die ein Arzneimittel im obigen Sinne aufgebracht ist und die dazu bestimmt sind, dauernd oder vorübergehend mit dem menschlichen oder tierischen Körper in Berührung gebracht zu werden, sowie Stoffe und Zubereitungen aus Stoffen, die, auch im Zusammenwirken mit anderen Stoffen oder Zubereitungen aus Stoffen, dazu bestimmt sind, ohne am oder im tierischen Körper angewendet zu werden, die Beschaffenheit, den Zustand oder die Funktion des tierischen Körpers erkennen zu lassen oder der Erkennung von Krankheitserregern bei Tieren zu dienen.

Die vorgenannte Aufgabe wird durch ein Hot-Melt-Coating-Verfahren gemäß Anspruch 1 gelöst, wobei sich vorteilhafte Ausführungsvarianten aus den Unteransprüchen ergeben.

Vorgeschlagen wird ein Hot-Melt-Coating-Verfahren, bei dem disperses Material mit einem Coatingmaterial zu einem Produkt aus oberflächenfesten Einzelteilen ummantelt wird, dadurch gekennzeichnet, dass das Coatingmaterial eine Zusammensetzung aufweist mit wenigstens 98 Gew.% eines oder mehrerer, jeweils aus einer vollständigen oder partiellen Veresterung eines zwei bis acht Glyceryleinheiten aufweisenden, linearen oder verzweigten Polyglycerols mit einer oder mehreren jeweils von 6 bis zu 22 Kohlenstoffatome aufweisenden Fettsäuren erhältlicher Polyglycerolfettsäureester als gewichtsprozentualer Hauptbestandteil, wobei das zu dem Versprühen des Coatingmaterials zeitgleich verwendete Gas oder Gasgemisch während des Versprühens eine Temperatur aufweist, die lediglich 1°C bis 3°C unterhalb der Erstarrungstemperatur des Coatingmaterials liegt.

Vorzugsweise besteht das vorgeschlagene Coatingmaterial, abgesehen von synthesebedingten Begleitstoffen, die bis zu zwei Gewichtsprozent ausmachen können, aus Polyglycerolfettsäureestern oder postsynthetischen Gemischen aus diesen.

Die einfachsten Polyglycerole, die als Ausgangsstoffe für die vorgesehene Veresterung eingesetzt werden können, sind lineare und verzweigte Diglycerole mit der Summenformel C₆O₅H₁₄, die industriell in bekannter Art und Weise synthetisch bereit gestellt werden, beispielsweise indem Glycerol mit 2,3-Epoxy-1-propanol basenkatalysiert unter Ausbildung von Etherbindungen umgesetzt oder basenkatalysiert thermal kondensiert wird, wobei die hauptsächlich Diglycerole enthaltende Fraktion nachträglich separiert werden kann.

Diglycerole können in drei verschiedenen strukturisomeren Formen auftreten, nämlich in der linearen Form, bei der die Etherbrücke zwischen den jeweils ersten Kohlenstoffatomen der beiden eingesetzten Glycerolmoleküle entsteht, in der verzweigten Form, bei die Etherbrücke zwischen dem ersten Kohlenstoffatom des ersten und dem zweiten Kohlenstoffatom des zweiten eingesetzten Glycerolmoleküls entsteht, und in einer nukleodentrimeren Form, bei der die Etherbrücke zwischen den jeweils zweiten Kohlenstoffatomen entsteht. Bei der alkalisch katalysierten Kondensation zweier Glycerolmoleküle entsteht etwa zu 80% die lineare Form und zu etwa 20% die verzweigte Form, während die nukleodentrimere Form nur in sehr geringem Maße gebildet wird.

Ebenso können für die vorgesehene Veresterung mit Fettsäuren Polyglyerole mit mehr als zwei und bis zu acht Glyceryleinheiten eingesetzt werden. Allgemein werden die Polyglycerole mit "PG" abgekürzt und mit einer tiefergestellten natürlichen Zahl n versehen, die die Anzahl der Polyglyceryleinheiten angibt, also "PGₙ". Beispielsweise würden Triglycerole als PG₃ angegeben und hätten die Summenformel C₉O₇H₂₀. Die vollständige Veresterung mit einer Fettsäure, zum Beispiel mit Stearinsäure, würde nun an allen freien Hydroxylgruppen des PGₙ-Moleküls stattfinden, im Falle eines linearen PG₃ also am ersten und zweiten Kohlenstoffatom der ersten Glyceryleinheit, am zweiten Kohlenstoffatom der zweiten Glyceryleinheit und am zweiten und dritten Kohlenstoffatom der dritten Glyceryleinheit. Die Summenformel für dieses Beispiel ließe sich daher mit C_{g}O₇H₁₅R₅ angeben, wobei R jeweils für einen Fettsäurerest stehen würde, im gewählten Beispiel mit der Summenformel C₁₈OH₃₅.

Für die Abkürzung von mit gesättigten unverzweigten Fettsäuren veresterten Polyglycerolen hat sich aber auch die Bezeichnung PG(n)-Cm-Vollester oder gegebenenfalls PG(n)-Cm-Partialester etabliert, wobei das eingeklammerte "n", ähnlich wie bei der Bezeichnung der Polyglycerole, die Anzahl der im Molekül enthaltenen Glyceryleinheiten angibt und m für die Anzahl der Kohlenstoffatome der für die Veresterungsreaktion verwendeten gesättigten Fettsäure steht. Das n steht also für die Anzahl an Glyceryleinheiten mit der Summenformel C₃O₂H₅R bzw. C₃O₃H₅R₂ für randständige Glyceryleinheiten, wobei R für einen Fettsäurerest oder das Wasserstoffatom einer freien Hydroxylgruppe stehen kann. PG(2)-C18-Vollester würde somit Polyglycerolfettsäurevollester mit der Summenformel C₇₈O₉H₁₅₀ bezeichnen. Im Falle der PG-Partialester wird die Anzahl der Fettsäurereste gemittelt, wobei die Summenformel zugleich die Fraktion mit den am häufigsten vorhandenen Veresterungsvarianten angibt. Eine genauere Bezeichnung für die Polyglycerolfettsäurepartialester ergibt sich durch die zusätzliche Angabe der Hydroxylzahl, die ein Maß für den Gehalt an unveresterten Hydroxylgruppen ist und damit Auskunft über den Veresterungsgrad des Partialesters gibt. Die Veresterungsreaktionen verlaufen dabei vorzugsweise, vermutlich aus sterischen Gründen, von außen nach innen. Es werden also zunächst die Hydroxylgruppen verestert, die dem Fettsäurerest die höchsten Freiheitsgrade erlauben. Die erste Veresterungsreaktion an einem linearen Polyglycerol findet demnach vorzugsweise an der Hydroxylgruppe des ersten Kohlenstoffatoms einer randständigen Polyglyceryleinheit statt, die zweite Veresterungsreaktion dann an der Hydroxylgruppe des dritten Kohlenstoffatoms der anderendseitig randständigen Polyglyceryleinheit. Im Weiteren werden die Hydroxylgruppen an bereits veresterten Positionen unmittelbar benachbarten Kohlenstoffpositionen verestert und so fort.

Unter Fettsäuren werden hier aliphatische Monocarbonsäuren mit 6 bis 22 Kohlenstoffatomen verstanden, die vorzugsweise unverzweigt und gesättigt sind und eine gerade Anzahl Kohlenstoffatome aufweisen, jedoch auch ungeradzahlig, verzweigt und/oder ungesättigt sein können. Besonders bevorzugt werden für die Bereitstellung der vorgeschlagenen Polyglycerolfettsäureester unverzweigte, gesättigte Fettsäuren mit 16, 18, 20 oder 22 C-Atomen eingesetzt, also Palmitin-, Stearin-, Arachin- oder Behensäure.

Überraschenderweise zeigen die vorgeschlagenen Polyglycerolfettsäureester im Gegensatz zu Monoglycerolfettsäureestern, wie beispielsweise Triacylglycerolen, keine Polymorphie. Die Polyglycerolfettsäureester weisen, jeweils einzeln mittels dynamischer Differenzkalorimetrie untersucht, für den Wärmefluss, der in mW/g angegeben wird, bei dem Aufwärmen jeweils lediglich ein endothermes Minimum auf, das aufgrund des Schmelzens der untersuchten Probe auftritt, und bei dem Abkühlen jeweils lediglich ein exothermes Maximum, das aufgrund der Verfestigung der untersuchten Probe auftritt. Im Gegensatz dazu zeigen sich bei der Untersuchung von Polymorphie aufweisenden Triacylglycerolen verschiedene lokale Minima, nämlich bei dem Aufwärmen der Probe ein erstes lokales endothermes Minimum bei dem Schmelzen der α-Modifikation, gefolgt von einem lokalen exothermen Maximum bei der Kristallisation zu der stabileren β-Modifikation, die angezeigt durch ein weiteres lokales endothermes Minimum des Wärmeflusses, bei weiter steigender Temperatur ebenfalls schmilzt. Die Temperaturänderungen beim Aufwärmen und beim Abkühlen erfolgen dabei gleichmäßig über die Zeit. Während einer Lagerung bei Raumtemperatur oder bei 40°C über 6 Monate werden keine zusätzlichen endo- oder exothermen Übergänge beobachtet.

Einzeluntersuchungen der vorgeschlagenen Polyglycerolfettsäureester unterhalb ihrer jeweiligen Erstarrungstemperatur mittels Weitwinkel-Röntgenstreuung, englisch "wide angle X-ray scattering", abgekürzt "WAXS", zeigen für alle untersuchten Polyglycerolfettsäureester ein Intensitätsmaximum, das auf einen Ablenkungswinkel von jeweils 21,4°, entsprechend etwa 2θ, also dem Zweifachen des Braggwinkels, schließen lässt, aus dem sich ein Abstand der Netzebenen von 415 pm ergibt, der hier mit der lamellaren Packungsdichte der untersuchten Moleküle korreliert. Dieser Abstand kann strukturell der α-Modifikation zugeordnet werden, bei der die jeweiligen lamellaren Strukturen in einem hexagonalen Gitter parallel zueinander angeordnet sind mit Ebenen bildenden, aufeinander gestapelten Molekülen. Andere Modifikationen lassen sich nicht identifizieren. Die Stabilität der identifizierten α-Modifikation wurde sowohl bei Raumtemperatur als auch bei 40°C über jeweils 6 Monate ebenfalls mittels WAXS betrachtet. Auch hierbei zeigte sich jeweils ausschließlich die für die untersuchten Polyglycerolfettsäureester überraschenderweise stabile α-Modifikation.

Eine weitere Bestätigung dafür, dass die vorgeschlagenen Polyglycerolfettsäureester keinen Polymorphismus aufweisen, ergibt die Einzelanalyse unterschiedlichster Polyglycerolfettsäureester mittels Kleinwinkel-Röntgenstreuung, englisch "small angle X-ray scattering", abgekürzt SAXS. SAXS erlaubt Rückschlüsse auf die Größe, die Form und die innere Oberfläche von Kristalliten. Die Dicke der jeweiligen Kristallite lässt sich dabei mittels der Scherrer-Formel berechnen, wonach D = Kλ / FWHM cos(θ) gilt. Dabei bezeichnet D die Dicke des Kristalliten und K die dimensionlose sogenannte Scherrer-Konstante, die Aussagen über die Form des Kristalliten erlaubt und im Regelfall in guter Näherung mit 0,9 angenommen werden kann. FWHM steht für englisch "full width at half maximum", also für die Breite des Peaks eines Intensitätsmaximums auf halber Höhe gegenüber dem Hintergrund gemessen im Bogenmaß (rad) und θ ist der Bragg-Winkel, also der Einfallswinkel der Strahlung auf die Netzebene. Während eine aus dem Stand der Technik bekannte Probe von mit 10% Polysorbat 65 stabilisiertem Glyceroltripalmitat nach sechsmonatiger Lagerung bei Raumtemperatur eine Kristallitendicke von 31 nm, entsprechend sieben Lamellen, aufweist und sich deren Kristallitendicke nach sechsmonatiger Lagerung bei 40°C mit 52 nm, entsprechend 12 Lamellen, nahezu verdoppelt, zeigen die vorgeschlagenen Polyglycerolfettsäurepartialester zumeist Kristallitendicken von 20 bis 30 nm, entsprechend 2 bis 4 Lamellen und sind nach sechsmonatiger Lagerung bei 40°C in unveränderter Modifikation stabil. Polyglycerolvollester zeigen demgegenüber zumeist eine auf einen höheren Organisationsgrad hinweisende, leicht erhöhte Kristallitendicke von 30 bis 40 nm, entsprechend 5 bis 8 Lamellen, und sind gleichfalls nach sechsmonatiger Lagerung bei 40° in unveränderter Modifikation stabil.

Bevorzugt für den Einsatz in oder als HMC-Coatingmaterial sind folgende Polyglycerolfettsäurevollester: PG(2)-C18, PG(2)-C22, PG(3)-C22, PG(4)-C16 sowie PG(4)-C16/C18 und PG(6)-C16/C18, jeweils mit einem sich zu 100 ergänzenden C16 zu C18-Verhältnis von 35 bis 45 zu 55 bis 65, vorzugsweise 40 zu 60. Vollester aus dieser Gruppe weisen Schmelzpunkte unter 80°C auf, abgesehen von PG(2)-C22 und PG(3)-C22 sogar unter 60°C, und sind daher ohne weiteres für HMC-Verfahren gut geeignet, zumal ihr für das Verfahren ebenfalls maßgeblicher Erstarrungspunkt in etwa 3°C bis 7°C unter dem jeweiligen Schmelzpunkt liegt. Gleiches gilt für folgende Polyglycerolfettsäurepartialester, wobei nachfolgend der jeweils bevorzugte Bereich für die typische mittlere Hydroxylzahl in der ersten eckigen Klammer und die jeweils besonders bevorzugte typische mittlere Hydroxylzahl in der zweiten eckigen Klammer an die Bezeichnung angehängt sind: PG(2)-C22-[15-100]-[17], PG(3)-C22-[100-200]-[137], PG(4)-C16-[150-250]-[186], PG(4)-C18-[100-200]-[168], PG(4)-C22-[100-200]-[145], PG(6)-C18-[100-200]-[133] sowie PG(3)-C16/C18-[100-200]-[148], PG(4)-C16/C18-[150-250]-[187], PG(6)-C16/C18-[200-300]-[237], jeweils mit einem C16 zu C18 Verhältnis von 40 zu 60. Die benannten Partialester weisen ebenfalls Schmelzpunkte unter 80°C auf, abgesehen von PG(2)-C22-Partialester, PG(3)-C22-Partialester und PG(4)-C22-Partialester sogar unter 60°C, wobei die Erstarrungspunkte, wie bei den Vollestern, in etwa 3°C bis 7°C unter den jeweiligen Schmelzpunkten liegen.

Damit die vorgeschlagenen Polyglycerolfettsäureester für ein HMC-Verfahren geeignet sind, sollte ihre Viskosität bei 80°C weniger als 300 mPa·s betragen, vorzugsweise weniger als 200 mPa·s und besonders bevorzugt weniger als 100 mPa·s, da die im Regelfall verwendeten Vernebelungsdüsen für das geschmolzene Coatingmaterial bei höheren Viskositäten zu leicht verstopfen würden. Die Grenze einer Schmelztemperatur des Coatingmaterials von 80°C sollte nur in Ausnahmefällen überschritten werden, da die Prozessführungstemperaturen dann insgesamt für empfindliche pharmazeutische Stoffe zu hoch angesetzt werden müssen.

Vorzugsweise kommen von den vorgeschlagenen Polyglycerolfettsäureestern diejenigen für ein HMC-Verfahren zum Einsatz, bei denen die Erstarrungstemperatur unter 75°C liegt, besonders bevorzugt zwischen 43°C und 56°C, da die damit möglichen niedrigen Prozessführungstemperaturen schon aus Gründen des Energieverbrauchs, der Prozesssicherheit und der größeren Auswahl bezüglich des einsetzbaren dispersen Materials anzustreben sind. Die Erstarrungstemperatur ist hier als der Temperaturwert definiert, an dem während einer Probenanalyse mittels dynamischer Differenzkalorimetrie bei dem Abkühlen das Maximum des höchsten exothermen Peaks des Wärmeflusses auftritt.

Für die Auswahl eines zu dem zu ummantelnden dispersen Material passenden vorgeschlagenen Polyglycerolfettsäureesters ist dessen Hydrophobizität von Bedeutung, da diese mit der Benetzbarkeit einher geht, die, ebenso wie die Wasseraufnahmefähigkeit und das Erosionsverhalten des Coatingmaterials, Einfluss auf die Freisetzungskinetik des ummantelten dispersen Materials hat. Die Bestimmung der Hydrophobizität erfolgt über die Bestimmung des Kontaktwinkels zwischen dem in festem Aggregatzustand befindlichen Coatingmaterial und einem Tropfen gereinigten Wassers. Gemäß der Gleichung nach Young gilt cosθ = (γ_{SV} - γ_{SL}) / γ_{LV}, wobei γ_{SL} die Grenzflächenspannung zwischen Coatingmaterial und Wasser, γ_{LV} die Oberflächenspannung des Wassertropfens und γ_{SV} die Grenzflächenspannung zwischen Coatingmaterial und Umgebungsluft angibt. θ ist der Kontaktwinkel. Je größer also der Kontaktwinkel θ, desto größer auch die Grenzflächenspannung zwischen Coatingmaterial und Wasser und desto höher die Hydrophobizität des untersuchten Coatingmaterials. Die Kontaktwinkel korrelieren für die vorgeschlagenen Polyglycerolfettsäureester auch mit dem in der pharmazeutischen Technologie gebräuchlichen HLB-Wert, der auf einer Skala von 0 bis 20 Auskunft über das Verhältnis lipophiler zu hydrophilen Molekülanteilen gibt, wobei der hydrophile Anteil mit steigendem HLB-Wert zunimmt. Der Kontaktwinkel des Coatingmaterials unter Lagerungsbedingungen sollte für die Verarbeitung einen oder mehrere pharmazeutische Wirkstoffe aufweisenden dispersen Materials mittels HMC-Verfahren nur moderaten Änderungen unterliegen, damit die Stabilität der Freisetzungskinetik des oder der pharmazeutischen Wirkstoffe gewährleistet ist. Bevorzugt werden daher von den Polyglycerolfettsäureestern solche als Hauptbestandteil des Coatingmaterials verwendet, deren Kontaktwinkel bei 40°C ebenso wie bei 20°C nach 16 Wochen weniger als 10° Abweichung vom Ausgangswert aufweist. Mit 40° vergleichsweise hoch und damit einer wünschenswerten Konstanz der Freisetzungskinetik abträglich wäre beispielsweise die Abweichung des Kontaktwinkels unter den benannten Bedingungen für Glyceroltristearin, die auf eine Umlagerung während der Lagerung von der α- in die β-Modifikation zurückzuführen ist.

Grundsätzlich ist es für die Bereitstellung eines vorgeschlagenen Coatingmaterials ausreichend, wenn dieses, abgesehen von synthesebedingten Verunreinigungen, die nicht mehr als zwei Gewichtsprozent betragen sollten, aus Polyglycerolfettsäureestern besteht, die aus Veresterungsreaktionen erhältlich sind, die sämtlich mit den gleichen Reaktionspartnern ausgeführt werden. Um Feinjustierungen der Eigenschaften des Coatingmaterials vorzunehmen ist es jedoch auch möglich, Polyglycerolfettsäureester postsynthetisch miteinander zu mischen, die aus aufgrund unterschiedlicher Reaktionspartner voneinander verschiedenen Veresterungsreaktionen erhältlich sind, soweit keine Entmischung auftritt. Auch neutrale Beimischungen zu den für das Coatingmaterial verwendeten Polyglycerolfettsäureestern sind möglich, soweit Polyglycerolfettsäureester Hauptbestandteil des Coatingmaterials bleiben, weder Polymorphismus noch Entmischungen auftreten, die Stabilität der Freisetzungskinetik gegeben ist und der Schmelz- und Erstarrungspunkt der Mischung unter 80°C liegt.

Damit weder vor noch nach dem HMC-Verfahren unerwünschte Entmischungsvorgänge auftreten, besteht das Coatingmaterial für das HMC-Verfahren bevorzugt zu wenigstens 98 Gewichtsprozent aus Polyglycerolfettsäureestern.

Im Gegensatz zu bereits bekannten Coatings oder Beschichtungsmitteln ist das vorgeschlagene Coatingmaterial vorzugsweise frei von Lösungsmitteln, die nach der Ummantelung des dipersen Materials in einem energie- und zeitaufwändigen Trocknungsschritt durch Verdampfen entfernt werden müssten. Vorteilhaft ist es auch, wenn das Coatingmaterial keine Tensidzusätze aufweist, da bei solchen Zusätzen häufig die Gefahr von unerwünschten Entmischungen besteht, die sich oft erst bei Langzeituntersuchungen zur Lagerungsstabilität zeigen.

Die Verwendung des vorgeschlagenen Coatingmaterials mit Polyglycerolfettsäureestern als Hauptbestandteil ist nicht auf das HMC-Verfahren beschränkt, solange es, mit welchem Verfahren auch immer, durch Versprühen seiner Schmelze in eine hohlkugelhomöomorphe Form gebracht wird, deren innerer Hohlraum oben beschriebenes disperses Material aufweist, wobei das disperse Material vorzugsweise wenigstens einen pharmazeutischen Wirkstoff aufweist. Überraschenderweise ist es mit dem vorgeschlagenen Coatingmaterial auch möglich, Kristalle eines oder mehrerer pharmazeutischen Wirkstoffe stabil zu ummanteln ohne die Notwendigkeit, zuvor ein Granulat oder Agglomerat mit einem oder mehreren Hilfsstoffen bereitstellen zu müssen.

Das beanspruchte Hot-Melt-Coating-Verfahren ergibt gegenüber dem Stand der Technik überlegene Endprodukte, die in ihrer Freisetzungskinetik gut justierbar sind und auch über längere Zeiträume ohne Qualitätsverlust gelagert werden können. Damit ist ein solches Hot-Melt-Coating-Verfahren prädestiniert dafür, auch wenigstens einen pharmazeutischen Wirkstoff aufweisendes disperses Material mit einem Schmelzüberzug zu versehen, insbesondere auch solches disperses Material, das wenigstens einen thermolabilen pharmazeutischen Wirkstoff aufweist, wobei thermolabil hier bedeutet, dass bereits nach einstündiger Exposition über 100°C die Wirkungsaktivität um 2% gemindert ist. 100 % Wirkungsaktivität eines pharmazeutischen Wirkstoffs liegen vor, wenn sämtliche Moleküle des pharmazeutischen Wirkstoffs in ihrer Wirkform vorliegen oder *in vivo* in diese überführt werden können.

Überraschenderweise konnte bei der Variation ergebniskritischer Parameter des HMC-Verfahrens, insbesondere der Lufteinlasstemperatur, festgestellt werden, dass diese für das vorgeschlagene Coatingmaterial nicht zwingend 5°C bis 15°C unter der Erstarrungstemperatur des Coatingmaterials liegen muss, sondern, möglicherweise aufgrund der gegenüber dem Stand der Technik geringeren spezifischen Wärmekapazität des vorgeschlagenen Coatingmaterials, bis zu 1°C bis 2°C an diese herangeführt werden kann und so effektiv die Bildung unerwünschter Agglomerate während des Sprühvorgangs verhindert.

Nachfolgend wird anhand von Abbildungen und eines Beispiels näher dargelegt, welche Eigenschaften das vorgeschlagene Coatingmaterial und Kombinationen aus dem Coatingmaterial und dispersem Material aufweisen und welche Parameter bei einem Hot-Melt-Coating-Verfahren, in dem das vorgeschlagene Coatingmaterial zum Einsatz kommt, auf welche Weise zu berücksichtigen sind.

In einer Glasapparatur mit Destillationsbrücke werden 595g PG₄ und 625g C18-Fettsäure vorgelegt und aufgeschmolzen. Die Reaktion wird unter Vakuum bei 200°C bis 240°C durchgeführt. Es wird so lange verestert, bis eine SZ <1,0 mg KOH/g erreicht ist.

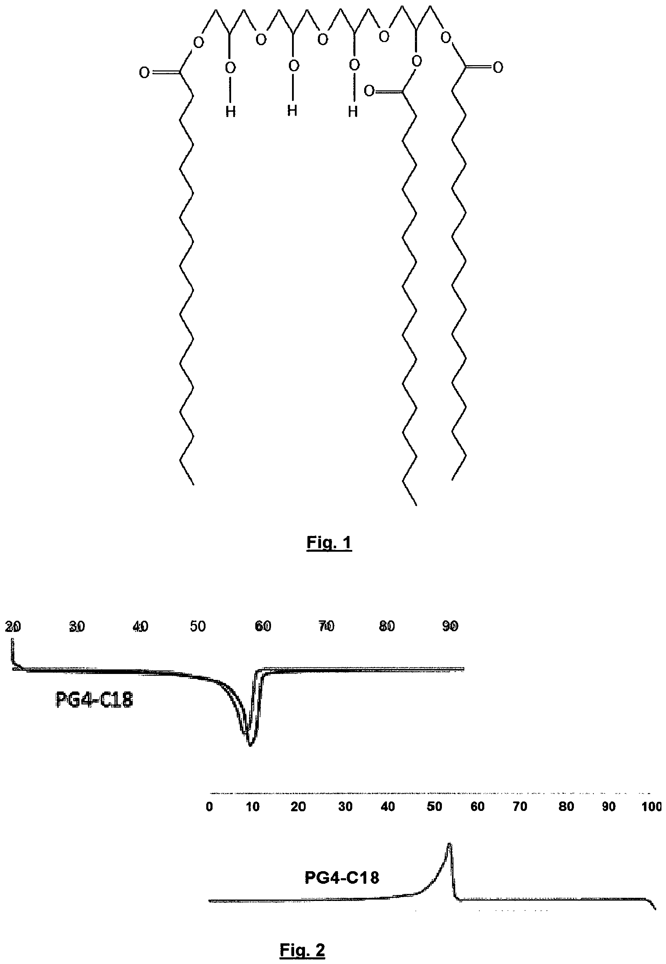

Der wie vorstehend synthetisierte Partialester PG(4)-C18 zeigt bei der Untersuchung mittels Gaschromatographie gekoppelt mit Massenspektroskopie (GC-MS) die in **Fig. 1** gezeigte mengenmäßige Hauptstruktur.

**Fig. 2** zeigt die Ergebnisse der Untersuchungen von PG(4)-C18 mittels dynamischer Differenzkalorimetrie, wobei die Temperaturwerte auf der X-Achse des Diagramms dem Wärmefluss in mW/g auf der Y-Achse zugeordnet sind. Das linke Diagramm in **Fig. 2** zeigt zwei nahezu deckungsgleiche Kurven zweier Messungen des Partialesters PG(4)-C18, die jeweils genau ein endothermes Minimum aufweisen, das dem energieverbrauchenden Übergang von der festen in die flüssige Phase beim Schmelzen des Partialesters zugeordnet werden kann. Das rechte Diagramm in **Fig. 2** zeigt für den Partialester PG(4)-C18 genau ein exothermes Maximum, das dem energiefreisetzenden Übergang von der flüssigen in die feste Phase beim Erstarren des Partialesters zugeordnet werden kann. Die Messungen wurden mit einem DSC 204 F1 Phoenix der Nietzsch Gerätebau GmbH, 95100 Selb, Deutschland, durchgeführt. Dabei wurde eine Probe von 3 - 4 mg in einen Aluminiumtiegel eingewogen und bei einer Erwärmungsrate von 5K pro Minute der Wärmefluss kontinuierlich erfasst. Ein zweiter Durchgang wurde mit derselben Erwärmungsrate durchgeführt.

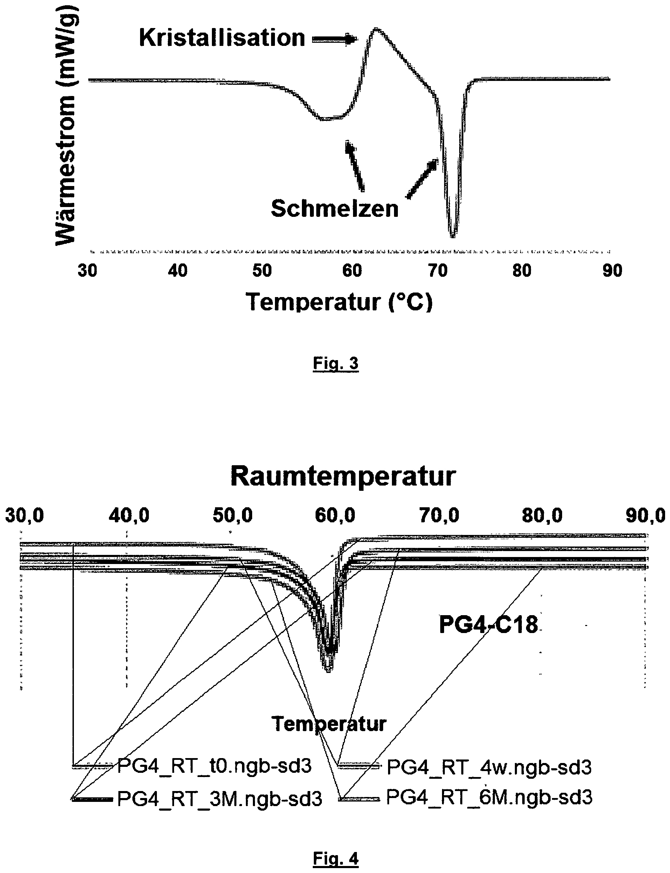

**Fig. 3** zeigt als Kontrast zu dem erwünschten Verhalten der Polyglycerolfettsäureester das typische Verhalten eines polymorphen Triacylglycerols während einer Untersuchung mittels dynamischer Differerenzkalorimetrie beim Aufwärmen. Hier sind zwei lokale endotherme Minima mit einem dazwischen liegenden exothermen Maximum zu erkennen, wobei das erste endotherme, linksseitige Minimum aufgrund des Schmelzens der instabilen α-Modifikation auftritt, gefolgt von dem exothermen Maximum bei der Kristallisation in die stabilere β-Modifikation, die wiederum bei weiterer Temperatursteigerung schmilzt, erkennbar durch das zweite endotherme, rechtsseitige lokale Minimum.

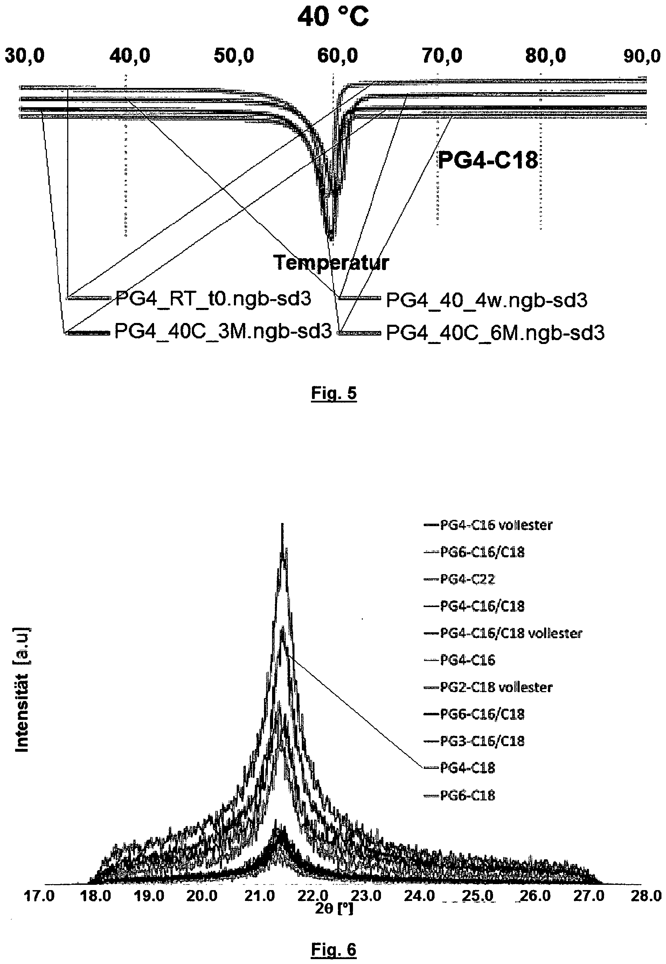

**Fig. 4** zeigt den mittels dynamischer Differenzkalorimetrie untersuchten PG(4)-C18-Partialester beim Aufwärmen nach 6-monatiger Lagerung bei Raumtemperatur. Fig. 5 zeigt den mittels dynamischer Differenzkalorimetrie untersuchten PG(4)-C18-Partialester beim Aufwärmen nach 6 monatiger Lagerung bei 40°C. In beiden Fällen zeigt sich nach wie vor kein exothermes Maximum, das auf die Kristallisation in eine stabilere Modifikation nach dem Schmelzen hindeuten könnte.

Für die WAXS- und die SAXS-Analysen wurde ein punktfokussierendes Kamerasystem, S3-MICRO, vormals Hecus X-ray Systems Gesmbh, 8020 Graz, Österreich, jetzt Bruker AXS GmbH, 76187 Karlsruhe, Deutschland, ausgestattet mit zwei linearen positionssensitiven Detektoren mit einer Auflösung von 3,3 - 4,9 Angström (WAXS) und 10 - 1500 Angström (SAXS) verwendet. Die Proben wurden in eine Glaskapillare von etwa 2 mm Durchmesser eingebracht, die nachträglich mit Wachs versiegelt und in der Kapillaren-Rotationseinheit platziert. Die Einzelmessungen wurden bei Raumtemperatur für 1300 sec einem Röntgenstrahl mit einer Wellenlänge von 1,542 Angström ausgesetzt.

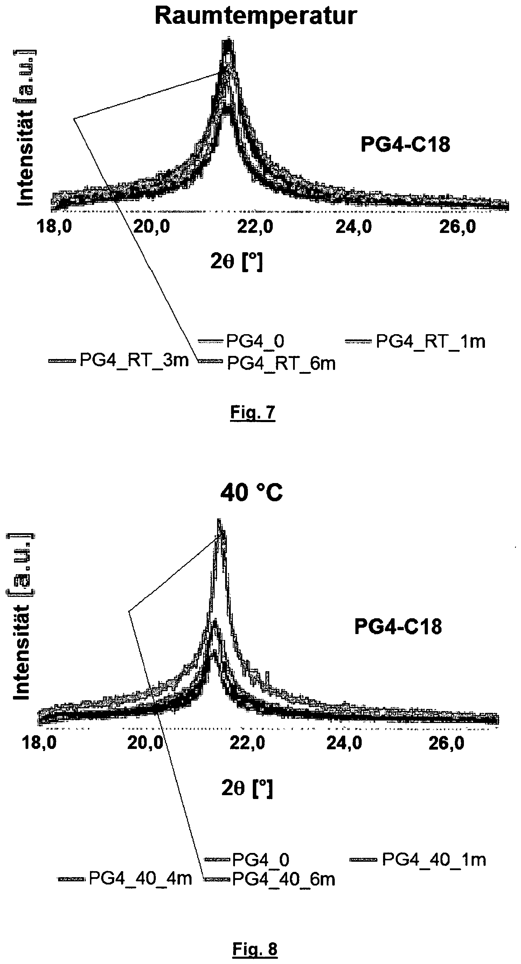

**Fig. 6** zeigt die Ergebnisse der WAXS-Analyse verschiedener Polyglycerolfettsäureester einschließlich PG(4)-C18-Partialester (markiert) unterhalb ihrer Erstarrungstemperatur, die alle ein Intensitätsmaximum bei 2 θ von 21,4° zeigen. Der Bragg-Winkel entspricht einem Abstand der Netzebenen von 415 pm, der typisch ist für die lamellare Packung der α-Modifikation. Das Intensitätsmaximum bleibt sowohl bei Lagerung über 6 Monate bei Raumtemperatur, wie in **Fig. 7** gezeigt, als auch bei Lagerung über 6 Monate bei 40°C stabil, wie in **Fig. 8** gezeigt.

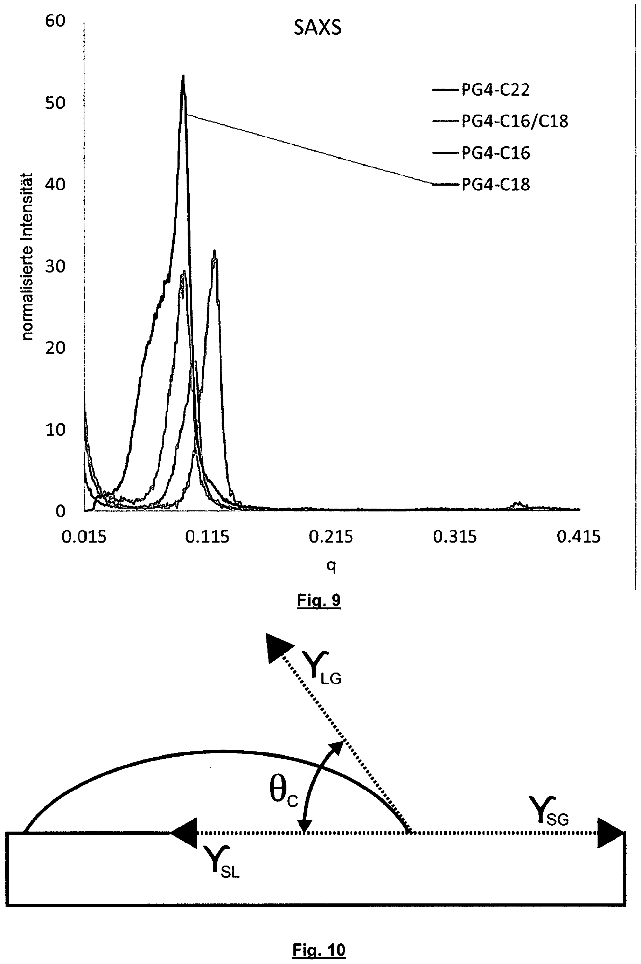

**Fig. 9** zeigt die Ergebnisse der SAXS-Analyse verschiedener Polyglycerolfettsäurepartialester. Für PG(4)-C18-Partialester lässt sich ein lamellarer Abstand von 65,2 Angström ableiten. Die Dicke der Kristalliten beträgt der Scherrer-Formel zufolge 12,5 nm, bei einer Scherrer-Konstanten von 0,9, einer Wellenlänge von 1,542 Angström, einem FWHM-Wert von 0,0111 und einem Bragg-Winkel θ von 0,047 (rad). Die Werte der SASX-Analyse von PG(4)-C18-Partialester blieben auch nach sechsmonatiger Lagerung sowohl bei Raumtemperatur als auch bei 40°C konstant (nicht gezeigt).

Für die Messung der Viskosität wurde ein Rheometer Physica - Modular Compact Rheometer, MCR 300 der Anton Paar Gmbh, 8054 Graz, Österreich, verwendet. Die Messung wurde auf einem CP-50-2-System mit konischer Platte bei konstanten Scherkräften durchgeführt. Dabei wurde die Probe direkt auf der Platte geschmolzen und die Viskosität bei 80°C und 100°C bestimmt. Die Viskosität für PG(4)-C18-Partialester beträgt demnach bei 80°C 74,38 mPa·s und bei 100°C 34,46 mPa·s. Der Partialester lässt sich somit sehr gut in einem Hot-Melt-Coating-Verfahren verarbeiten.

Die Auswertung der dynamischen Differenzkalorimetrie erlaubt auch Aussagen über die Erstarrungstemperatur des PG(4)-C18-Partialesters. Der Peak des exothermen Maximums bei dem Abkühlen der Probe erhebt sich zwischen 53,4°C und 57,0°C mit dem Maximum bei 55,2°C, das die Erstarrungstemperatur markiert.

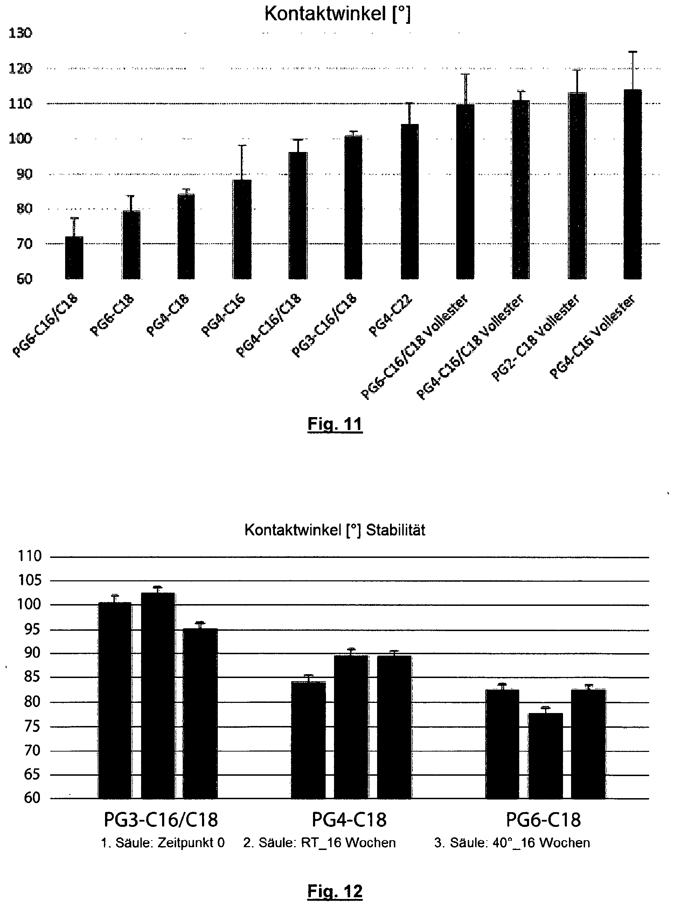

**Fig. 10** zeigt ein Schema, das die Messung des Kontaktwinkels verdeutlicht (vgl. Abs. [0020]). Für PG(4)-C18-Partialester beträgt der Kontaktwinkel etwa 84°, was mit einem HLB-Wert von etwa 5,2 korreliert. Im Vergleich zu weiteren Polyglycerolfettsäureestern ist PG(4)-C18-Partialester den hydrophileren Polyglycerolfettsäureestern zuzuordnen, wie aus **Fig. 11** ersichtlich (dort = PG4-C18), und damit für die Ummantelung von pharmazeutischen Wirkstoffen geeignet, für die eine sofortige Freisetzung erwünscht ist, da der HLB-Wert mit 5,2 über dem HLB-Grenzwert für schnelle Freisetzung von etwa 4 liegt. **Fig. 12** zeigt die Veränderung des Kontaktwinkels für PG(4)-C18-Partialester, mittiges Diagramm, gegenüber der Startmessung (linke Säule) nach 16 Wochen bei Raumtemperatur (mittlere Säule) und nach 16 Wochen bei 40°C (rechte Säule). Der Kontaktwinkel ändert sich um nicht mehr als 10°, die Hydrophobizität kann somit im Vergleich mit Monoglycerolfettsäureestern, wie beispielsweise Tristearylglycerol, als stabil bezeichnet werden. Gleiches gilt für die ebenfalls in **Fig. 12** gezeigten PG3-C16/C18-Partialester, linkes Diagramm, und PG6-C18-Partialester, rechtes Diagramm.

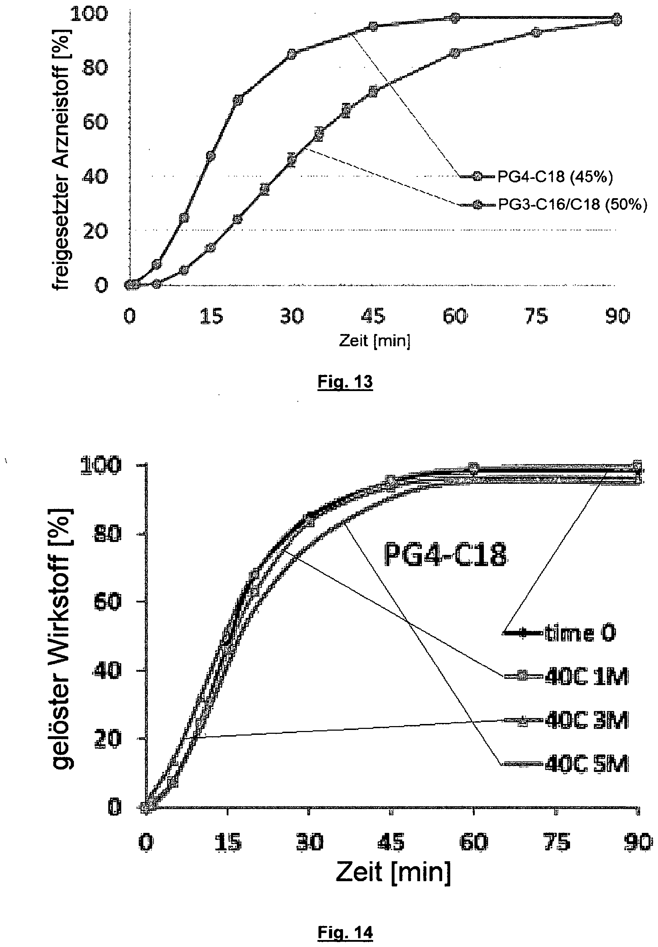

**Fig. 13** zeigt die Freisetzungskinetiken für mit PG(4)-C18-Partialester und alternativ mit PG(3)-C16/C18-Partialester ummmanteltem Partikeln mit je 600 mg N-Acetylcystein. Der Anteil PG(4)-C18-Partialesters betrug 45 %, der Anteil PG(3)-C16/C18-Partialesters 50% des Gesamtgewichts der ummantelten Partikeln. Die Werte auf der Y-Achse stehen für die prozentualen Anteile des freigesetzten N-Acetylcysteins, die Werte auf der X-Achse für die Zeit in Minuten. Die Freisetzungsuntersuchungen wurden mit einer USP-II-konformen Vorrichtung, einem DT820LH der ERWEKA GmbH, 63150 Heusenstamm, Deutschland, durchgeführt, das über einen automatischen Probensammler verfügt. Die gesammelten Proben wurden mittels Hochdruckflüssigkeitschromatographie, englisch "high pressure liquid chromatography", abgekürzt "HPLC", unter folgenden Bedingungen analysiert: Säule: Synergi Fusio RP 4 µm, 80 Angström, 250 mm x 4,6 mm; vorgeschaltete Säule: Atlantis T3 (5 µm); mobile Phase: Acetonitril 5% / Wasser 95% (pH 1,6); Flussrate: 1 mL/min; Injektionsvolumen: 20 µm; Säulentemperatur: 21°C; Temperatur des automatischen Probensammlers: 5°C; Wellenlänge: 220 nm; Laufzeit: 20 min. Die mit PG(4)-C18-Partialester ummantelten Partikeln weisen ein sofort freisetzendes Profil auf, bei dem innerhalb der ersten 5 Minuten weniger als 10% und innerhalb der ersten 30 Minuten mehr als 85% des N-Acetylcysteins freigesetzt werden. Um eine effektivere Geschmacksmaskierung zu erreichen, kann das eingesetzte HMC-Verfahren noch mit höherer Temperatur der verwendeten Einlassluft und höheren Sprühraten gefahren werden, um die Freisetzung des N-Acetylcysteins innerhalb der ersten 5 Minuten weiter abzusenken. Als gelungen kann die Geschmacksmaskierung aufgrund der Freisetzungskinetik der mit PG(3)-C16/C18-Partialester ummantelten Partikeln bezeichnet werden. Hier findet in den für die Geschmacksmaskierung kritischen ersten 5 Minuten so gut wie keine Freisetzung statt.

**Fig. 14** zeigt die Freisetzungskinetiken der mit PG(4)-C18-Partialester ummantelten N-Acetylcystein-Partikeln zu Beginn, nach einmonatiger, dreimonatiger und fünfmonatiger Lagerung bei 40°C. Die Freisetzungskinetiken unterscheiden sich nicht nennenswert, das Produkt ist stabil.

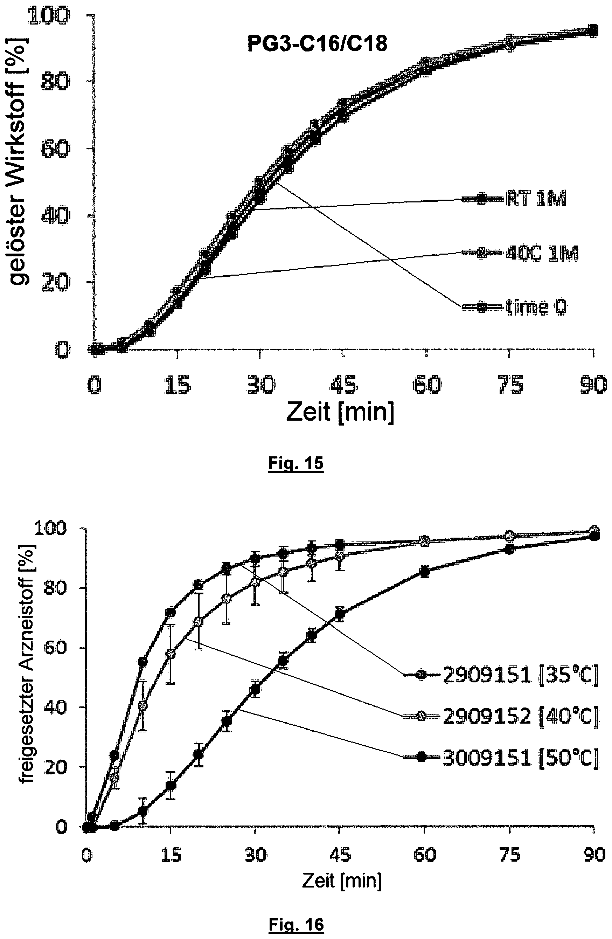

**Fig. 15** zeigt die Freisetzungskinetiken der mit PG(3)-C16/C18-Partialester ummantelten N-Acetylcystein-Partikeln zu Beginn, nach einmonatiger Lagerung bei Raumtemperatur und nach einmonatiger Lagerung bei 40°C. Auch hier unterscheiden sich die Freisetzungskinetiken nicht nennenswert.

Die gelungene Geschmacksmaskierung durch hauptanteilig PG(3)-C16/C18-Partialester aufweisendes Coatingmaterial konnte nicht zuletzt durch die Optimierung der HMC-Prozessparameter erreicht werden. Als Coating-Vorrichtung diente ein Innojet Ventilus V-2,5/1 Laborsystem in Kombination mit der Innojet-Hotmelt-Vorrichtung IHD-1 der Romaco Holding GmbH, 76227 Karlsruhe, Deutschland. PG(3)-C16/C18-Partialester wurde bei 100°C geschmolzen und auf N-Acetylcystein-Kristalle mit einem mittleren Durchmesser von etwa 500µm gesprüht. Die Probengröße für die HMC-Durchläufe betrug jeweils 200 g dispersen Materials. Bei den verschiedenen HMC-Durchläufen wurden die Sprühraten und die Lufteinlasstemperaturen verändert, um die für die Ummantelung optimale Einstellung zu ermitteln. Die Effektivität des Ummantelungsprozesses wurde dabei nach folgender Gleichung bestimmt: Effektivität (%) = tatsächliche Ummantelungsmenge / theoretisch erreichbare Ummantelungsmenge x 100, wobei die tatsächliche Ummantelungsmenge der im jeweiligen HMC-Durchlauf auf die Acetylcysteinkristalle aufgebrachte prozentuale Anteil des eingesetzten Coatingmaterials ist. Bei einer Sprayrate von 5 g/min und einer Lufteinlasstemperatur von 35,0°C betrug die Effektivität 90,7%. Eine Erhöhung der Lufteinlasstemperatur auf 40,0°C steigerte die Effektivität auf 91,0%. Eine Erhöhung der Sprayrate auf 7,5 g/min und der Lufteinlasstemperatur auf 50°C ergab überraschenderweise eine Effektivität von 100%. Die beiden Effektivitätswerte von 90,7% und 91,0% bedeuten, dass 9,3 bzw. 9,0 Gewichtsprozent des Coatingmaterials erstarrt sind bevor eine Ausbreitung und Verteilung auf der Oberfläche der N-Acetylcysteinkristalle stattfinden kann. Die wirkstofffreien erstarrten Tröpfchen wurden am Ende des jeweiligen Durchlaufs als Staub gesammelt und gewogen. Im Falle der 90,7%igen Effektivität waren es 18,6 g, im Falle der 91,0%igen Effektivität 18,0 g. Die Effektivität von 100% wurde dabei mit einer Lufteinlasstemperatur von weniger als 2°C unter der Erstarrungstemperatur des Coatingmaterials erreicht, in diesem Fall des PG(3)-C16/C18-Partialesters, der eine Erstarrungstemperatur von 51,7°C aufweist. Die gegenüber herkömmlichen HMC-Coatings niedrige spezifische Wärmekapazität der für das vorgeschlagene Coatingmaterial eingesetzten Polyglycerolfettsäureester mag ein Grund dafür sein, dass eine gegenüber dem Stand der Technik größere, vorteilhafte Flexibilität bei der Einstellung der Lufteinlasstemperatur nunmehr möglich ist. In der Publikation "Solvent-free melting techniques for the preparation of lipid-based solid oral formulations", K. Becker et al. in Pharmaceutical Research, May 2015, 32(5), 1519-45, werden noch Lufteinlasstemperaturen von 5°C bis 15°C unterhalb der Erstarrungstemperatur des HMC-Coatingmaterials für unabdingbar gehalten.

Im Unterschied zu den Freisetzungstests mit PG(4)-C18-Partialester aufweisendem Coatingmaterial wurde zur Ermittlung der Freisetzungskinetik der mit PG(3)-C16/C18-Partialester aufweisendem Coatingmaterial ummantelten N-Acetylcystein-Kristalle statt eines automatischen Probensammlers eine integrierte Detektion für UV-Strahlung/sichtbares Licht mit einem Lambda 25 Spektrometer der Perkin Elmer Inc., Waltham, Massachusetts, USA, verwendet. Die Freisetzungstests wurden bei 37°C in 900 mL ultrareinen Wassers, bezogen von Merck KGaA, Darmstadt, Deutschland, bei einer Paddelrührgeschwindigkeit von 100 Umdrehungen pro Minute durchgeführt. Die Freisetzungsprofile wurden zu Beginn auf 0% gesetzt und auf 100% Freisetzung für das Freisetzungslevel am Ende der Freisetzung skaliert. **Fig. 16** zeigt die Freisetzungskurven für die mit einer Lufteinlasstemperatur von 35°C, 40°C und 50°C im HMC-Verfahren ummantelten Partikeln. Die bei 50°C mit einer Effektivität von 100% ummantelten Partikeln weisen innerhalb der ersten 5 Minuten so gut wie keine Freisetzung von N-Acetylcystein auf. Das angewandte HMC-Verfahren mit dem PG(3)-C16/C18-Partialester als Hauptbestandteil aufweisenden Coatingmaterial ist somit nach mit dem vorgeschlagenen Coatingmaterial in dieser Weise überraschend möglichen Optimierung der Lufteinlasstemperatur zur Geschacksmaskierung gut geeignet.

## Patentansprüche

1. Hot-Melt-Coating-Verfahren, bei dem disperses Material mit einem Coatingmaterial zu einem Produkt aus oberflächenfesten Einzelteilen ummantelt wird,
**dadurch gekennzeichnet,**
**dass** das Coatingmaterial eine Zusammensetzung aufweist mit wenigstens 98 Gew.% eines oder mehrerer, jeweils aus einer vollständigen oder partiellen Veresterung eines zwei bis acht Glyceryleinheiten aufweisenden, linearen oder verzweigten Polyglycerols mit einer oder mehreren jeweils von 6 bis zu 22 Kohlenstoffatome aufweisenden Fettsäuren erhältlicher Polyglycerolfettsäureester als gewichtsprozentualer Hauptbestandteil, wobei das zu dem Versprühen des Coatingmaterials zeitgleich verwendete Gas oder Gasgemisch während des Versprühens eine Temperatur aufweist, die lediglich 1°C bis 3°C unterhalb der Erstarrungstemperatur des Coatingmaterials liegt.

2. Hot-Melt-Coating-Verfahren nach Anspruch 1,
**dadurch gekennzeichnet,**
**dass** die Fettsäuren, auf denen der oder die Polyglycerolfettsäureester des Coatingmaterials basieren, gesättigt oder unverzweigt oder sowohl gesättigt als auch unverzweigt sind.

3. Hot-Melt-Coating-Verfahren nach einem der vorhergehenden Ansprüche,
**dadurch gekennzeichnet,**
**dass** die Fettsäuren, auf denen der oder die Polyglycerolfettsäureester des Coatingmaterials basieren, 16, 18, 20 oder 22 Kohlenstoffatome aufweisen.

4. Hot-Melt-Coating-Verfahren nach einem der vorhergehenden Ansprüche,
**dadurch gekennzeichnet,**
**dass** die Untersuchung des oder der einzelnen Polyglycerolfettsäureester des Coatingmaterials mittels dynamischer Differenzkalorimetrie für den Wärmefluss bei dem Aufwärmen jeweils lediglich ein endothermes Minimum und bei dem Abkühlen jeweils lediglich ein exothermes Maximum ergibt.

5. Hot-Melt-Coating-Verfahren nach einem der vorhergehenden Ansprüche,
**dadurch gekennzeichnet,**
**dass** der oder die Polyglycerolfettsäureester des Coatingmaterials eine unterhalb der ihrer Erstarrungstemperatur stabile subzelluläre Form aufweisen mit bei 40°C über wenigstens 6 Monate im Wesentlichen konstantem lamellarem Abstand gemäß Auswertung des mittels WAXS-Analyse ermittelten Bragg-Winkels.

6. Hot-Melt-Coating-Verfahren nach einem der vorhergehenden Ansprüche,
**dadurch gekennzeichnet,**
**dass** der oder die Polyglycerolfettsäureester des Coatingmaterials eine unterhalb der ihrer Erstarrungstemperatur stabile subzelluläre Form aufweisen mit bei 40°C über wenigstens 6 Monate im Wesentlichen konstanter Dicke der lamellar strukturierten Kristallite gemäß mittels Scherrer-Formel ausgewerteter SAXS-Analyse.

7. Hot-Melt-Coating-Verfahren nach einem der vorhergehenden Ansprüche,
**gekennzeichnet durch**
wenigstens einen Polyglycerolfettsäureester des Coatingmaterials aus der folgenden Gruppe:
PG(2)-C18-Vollester, PG(2)-C22-Partialester mit einer Hydroxylzahl von 15 bis 100, PG(2)-C22-Vollester, PG(3)-C16/C18-Partialester mit einer Hydroxylzahl von 100 bis 200, PG(3)-C22-Partialester mit einer Hydroxylzahl von 100 bis 200, PG(3)-C22-Vollester, PG(4)-C16-Partialester mit einer Hydroxylzahl von 150 bis 250, PG(4)-C16-Vollester, PG(4)-C16/C18-Partialester mit einer Hydroxylzahl von 150 bis 250, PG(4)-C16/C18-Vollester, PG(4)-C18-Partialester mit einer Hydroxylzahl von 100 bis 200, PG(4)-C22-Partialester mit einer Hydroxylzahl von 100 bis 200, PG(6)-C16/C18-Partialester mit einer Hydroxylzahl von 200 bis 300, PG(6)-C16/C18-Vollester, PG(6)-C18-Partialester mit einer Hydroxylzahl von 100 bis 200, wobei in den Polyglycerolfettsäureestern mit zwei aufgrund der Anzahl ihrer Kohlenstoffatome verschiedenen Fettsäureresten, diejenigen mit der geringeren Anzahl zu 35% bis 45%, diejenigen mit der höheren Anzahl entsprechend komplementär zu 55% bis 65% vorliegen und die aufgeführten Vollester vorzugsweise eine Hydroxylzahl kleiner 5 aufweisen.

8. Hot-Melt-Coating-Verfahren nach einem der vorhergehenden Ansprüche,
**gekennzeichnet durch**
eine Viskosität des oder der Polyglycerylfettsäureester des Coatingmaterials bei 80°C kleiner 300 mPa·s.

9. Hot-Melt-Coating-Verfahren nach einem der vorhergehenden Ansprüche,
**gekennzeichnet durch**
eine Erstarrungstemperatur des oder der einzelnen Polyglycerolfettsäureester des Coatingmaterials unter 75°C.

10. Hot-Melt-Coating-Verfahren nach einem der vorhergehenden Ansprüche,
**dadurch gekennzeichnet,**
**dass** der zur Bestimmung der Hydrophobizität bestimmte Kontaktwinkel des oder der einzelnen Polyglycerolfettsäureester des Coatingmaterials bei 40°C ebenso wie bei 20°C nach 16 Wochen weniger als 10° Abweichung vom Ausgangswert aufweist.

11. Hot-Melt-Coating-Verfahren nach einem der vorhergehenden Ansprüche,
**gekennzeichnet durch**
eine postsynthetische Mischung aus Polyglycerolfettsäureestern als gewichtsprozentualer Hauptbestandteil des Coatingmaterials, die aus jeweils aufgrund der eingesetzten Reakionspartner voneinander verschiedenen Veresterungsreaktionen erhältlich sind.

12. Hot-Melt-Coating-Verfahren nach einem der vorhergehenden Ansprüche,
**gekennzeichnet durch**
eine lösungsmittelfreie oder tensidzusatzfreie oder sowohl lösungsmittel- als auch tensidzusatzfreie Zusammensetzung des Coatingmaterials.

13. Hot-Melt-Coating-Verfahren nach einem der vorhergehenden Ansprüche,
**dadurch gekennzeichnet,**
**dass** das disperse Material wenigstens einen pharmazeutischen Wirkstoff aufweist.

14. Hot-Melt-Coating-Verfahren nach einem der vorhergehenden Ansprüche,
**dadurch gekennzeichnet,**
**dass** das disperse Material aus Kristallen eines oder mehrerer pharmazeutischer Wirkstoffe besteht.

15. Hot-Melt-Coating-Verfahren nach einem der Ansprüche 13 oder 14,
**dadurch gekennzeichnet,**
**dass** wenigstens einer der pharmazeutischen Wirkstoffe thermolabil ist und nach der Ummantelung und nachfolgender Abkühlung auf Raumtemperatur mehr als 98% seiner ursprünglichen Wirkungsaktivität aufweist.

## Claims

1. A hot-melt coating method, in which disperse material is coated with a coating material to form a product consisting of surface-stable individual parts,
**characterized in that**
the coating material has a composition with
at least 98 % by weight of one or more polyglycerol fatty acid esters each obtainable by complete or partial esterification of a linear or branched polyglycerol containing two to eight glycerol units with one or more fatty acid, each containing 6 to 22 carbon atoms, as the main constituent as a percentage by weight, wherein the gas or gas mixture used simultaneously to the spraying of the coating material has a temperature only 1°C to 3°C below the solidification temperature of the coating material during the spraying.

2. The hot-melt coating method as claimed in claim 1,
**characterized in that**
the fatty acids on which the polyglycerol fatty acid ester or polyglycerol fatty acid esters of the coating material are based are saturated, unbranched or both saturated and unbranched.

3. The hot-melt coating method as claimed in one of the preceding claims,
**characterized in that**
the fatty acids on which the polyglycerol fatty acid ester or polyglycerol fatty acid esters of the coating material are based contain 16, 18, 20 or 22 carbon atoms.

4. The hot-melt coating method as claimed in one of the preceding claims,
**characterized in that**
an examination of the polyglycerol fatty acid ester or the individual polyglycerol fatty acid esters of the coating material using dynamic differential calorimetry results for the heat flux in only one endothermic minimum during heating and only one exothermic maximum during cooling.

5. The hot-melt coating method as claimed in one of the preceding claims,
**characterized in that**
the polyglycerol fatty acid ester or polyglycerol fatty acid esters of the coating material have a stable subcellular form below their solidification temperature with a substantially constant lamellar spacing at 40°C for at least 6 months as determined by analysis of the Bragg angle using WAXS analysis.

6. The hot-melt coating method as claimed in one of the preceding claims,
**characterized in that**
the polyglycerol fatty acid ester or polyglycerol fatty acid esters of the coating material have a stable subcellular form below their solidification temperature with a substantially constant thickness of the lamellar structured crystallites for at least 6 months at 40°C, as evaluated using SAXS analysis with the Scherrer formula.

7. The hot-melt coating method as claimed in one of the preceding claims,
**characterized by**
at least one polyglycerol fatty acid ester of the coating material from the following group:
PG(2)-C18 full esters, PG(2)-C22 partial esters with a hydroxyl value of 15 to 100, PG(2)-C22 full esters, PG(3)-C16/C18 partial esters with a hydroxyl value of 100 to 200, PG(3)-C22 partial esters with a hydroxyl value of 100 to 200, PG(3)-C22 full esters, PG(4)-C16 partial esters with a hydroxyl value of 150 to 250, PG(4)-C16 full esters, PG(4)-C16/C18 partial esters with a hydroxyl value of 150 to 250, PG(4)-C16/C18 full esters, PG(4)-C18 partial esters with a hydroxyl value of 100 to 200, PG(4)-C22 partial esters with a hydroxyl value of 100 to 200, PG(6)-C16/C18 partial esters with a hydroxyl value of 200 to 300, PG(6)-C16/C18 full esters, PG(6)-C18 partial esters with a hydroxyl value of 100 to 200, wherein in the polyglycerol fatty acid esters containing two fatty acid residues which are different because of the number of their carbon atoms, those with the lower number are present in an amount of 35 % to 45 %, those with the higher number are present in a complementary amount of 55 % to 65 %, and the specified full esters preferably have a hydroxyl value less than 5.

8. The hot-melt coating process as claimed in one of the preceding claims,
**characterized by**
a viscosity of the polyglycerol fatty acid ester or polyglycerol fatty acid esters of the coating material of less than 300 mPa·s at 80°C.

9. The hot-melt coating method as claimed in one of the preceding claims,
**characterized by**
a solidification temperature of the polyglycerol fatty acid ester or individual polyglycerol fatty acid esters of the coating material which is below 75°C.

10. The hot-melt coating method as claimed in one of the preceding claims,
**characterized in that**
the contact angle of the polyglycerol fatty acid ester or individual polyglycerol fatty acid esters of the coating material during the determination of the hydrophobicity shows less than a 10° deviation from the initial value after 16 weeks both at 40°C and 20°C.

11. The hot-melt coating method as claimed in one of the preceding claims,
**characterized by**
a post-synthetic mixture of polyglycerol fatty acid esters as the main constituent of the coating material as a percentage by weight which are obtainable from esterification reactions which respectively differ from one another due to the reaction partners employed.

12. The hot-melt coating method as claimed in one of the preceding claims,
**characterized by**
a composition of the coating material that is solvent-free, surfactant-free or both solvent-free and surfactant-free.

13. The hot-melt coating method as claimed in one of the preceding claims,
**characterized in that**
the dispersed material contains at least one active pharmaceutical ingredient.

14. The hot-melt coating method as claimed in one of the preceding claims,
**characterized in that**
the disperse material consists of crystals of one or more active pharmaceutical ingredients.

15. The hot-melt coating method as claimed in claim 13 or claim 14,
**characterized in that**
at least one of the active pharmaceutical ingredients is thermolabile and, after coating and subsequent cooling to room temperature, has more than 98% of its original effective activity.

## Revendications

1. Procédé de revêtement thermofusible, lors duquel l'on enrobe une matière dispersée d'une matière de revêtement en un produit en pièces détachées à surface solide,
**caractérisé**
**en ce que** la matière de revêtement présente une composition d'au moins 98 % en poids d'un ou de plusieurs esters d'acides gras de polyglycérol, chacun susceptible d'être obtenu par une estérification totale ou partielle d'un polyglycérol linéaire ou ramifié, comportant de deux à huit unités de glycéryle avec un ou plusieurs acide(s) gras comportant chacun de 6 à 22 atomes de carbone au titre de composant principal en pourcentage en poids, le gaz ou le mélange gazeux utilisé simultanément avec la pulvérisation de la matière de revêtement présentant pendant la pulvérisation une température qui est seulement inférieure de 1°C à 3°C à la température de solidification de la matière de revêtement.

2. Procédé de revêtement thermofusible selon la revendication 1,
**caractérisé**
**en ce que** les acides gras, sur lesquels sont basés le ou les esters d'acides gras de polyglycérol de la matière de revêtement sont saturés ou non ramifiés ou aussi bien saturés que non ramifiés.

3. Procédé de revêtement thermofusible selon l'une quelconque des revendications précédentes,
**caractérisé**
**en ce que** les acides gras, sur lesquels sont basés le ou les esters d'acides gras de polyglycérol de la matière de revêtement comportent 16, 18, 20 ou 22 atomes de carbone.

4. Procédé de revêtement thermofusible selon l'une quelconque des revendications précédentes,
**caractérisé**
**en ce que** l'analyse du ou des esters d'acides gras de polyglycérol individuels de la matière de revêtement par calorimétrie différentielle pour le flux thermique donne lieu lors de l'échauffement à chaque fois seulement un minimum endothermique et lors du refroidissement à chaque fois seulement un maximum exothermique.

5. Procédé de revêtement thermofusible selon l'une quelconque des revendications précédentes,
**caractérisé**
**en ce que** le ou les esters d'acides gras de polyglycérol de la matière de revêtement présentent une forme subcellulaire stable en-dessous de leur température de solidification, avec un intervalle lamellaire sensiblement constant à 40°C sur au moins 6 mois selon l'évaluation de l'angle de Bragg déterminée par l'analyse WAXS.

6. Procédé de revêtement thermofusible selon l'une quelconque des revendications précédentes,
**caractérisé**
**en ce que** le ou les esters d'acides gras de polyglycérol de la matière de revêtement présentent une forme subcellulaire stable en-dessous de leur température de solidification, avec une épaisseur lamellaire sensiblement constante à 40°C sur au moins 6 mois des cristallites à structure lamellaire selon l'analyse SAXS évaluée par la formule de Scherrer.

7. Procédé de revêtement thermofusible selon l'une quelconque des revendications précédentes,
**caractérisé par**
au moins un ester d'acides gras de polyglycérol de la matière de revêtement du groupe suivant :
ester complet PG(2) en C₁₈, ester partiel PG(2) en C₂₂ avec un indice d'hydroxyle de 15 à 100, ester complet PG(2) en C₂₂, ester partiel PG(3) en C₁₆ / C₁₈ avec un indice d'hydroxyle de 100 à 200, ester partiel PG(3) en C₂₂ avec un indice d'hydroxyle de 100 à 200, ester complet PG(3) en C₂₂, ester partiel PG(4) en C₁₆ avec un indice d'hydroxyle de 150 à 250, ester complet PG(4) en C₁₆, ester partiel PG(4) en C₁₆/C₁₈ avec un indice d'hydroxyle de 150 à 250, ester complet PG(4)en C₁₆ / C₁₈, ester partiel PG(4) en C₁₈ avec un indice d'hydroxyle de 100 à 200, ester partiel PG(4) en C₂₂, avec un indice d'hydroxyle de 100 à 200, ester partiel PG(6) en C₁₆/ C₁₈ avec un indice d'hydroxyle de 200 à 300, ester complet PG(6) en C₁₆/C₁₈, ester partiel PG(6)en C₁₈ avec un indice d'hydroxyle de 100 à 200, dans les esters d'acides gras de polyglycérol avec deux esters d'acides gras différents du fait du nombre de leurs atomes de carbone, ceux du plus faible nombre étant présents à raison de 35 % à 45 %, ceux du nombre le plus élevé étant présents en conséquence de manière complémentaire à raison de 55 % à 65 % et les esters complets mentionnés faisant preuve de préférence d'un indice d'hydroxyle inférieur à 5.

8. Procédé de revêtement thermofusible selon l'une quelconque des revendications précédentes,
**caractérisé par**
une viscosité à 80 °C de ou des ester(s) d'acides gras de polyglycérol de la matière de revêtement qui est inférieure à 300 mPa*s.

9. Procédé de revêtement thermofusible selon l'une quelconque des revendications précédentes,
**caractérisé par**
une température de solidification du ou des ester(s) d'acides gras de polyglycérol individuels de la matière de revêtement qui est inférieur à 75 °C.

10. Procédé de revêtement thermofusible selon l'une quelconque des revendications précédentes,
**caractérisé**
**en ce que** l'angle de contact du ou des ester(s) d'acides gras de polyglycérol individuels de la matière de revêtement déterminé pour la détermination de l'hydrophobie présente à 40 °C, tout comme à 20 °C après 16 semaines un écart de moins de 10° par rapport à la valeur initiale.

11. Procédé de revêtement thermofusible selon l'une quelconque des revendications précédentes,
**caractérisé par**
un mélange post-synthétique d'esters d'acides gras de polyglycérol, au titre de composant principal en pourcentage en poids de la matière de revêtement, qui sont susceptibles d'être obtenus à partir de réactions d'estérification différentes l'une de l'autre, respectivement du fait des partenaires de réaction mis en oeuvre.

12. Procédé de revêtement thermofusible selon l'une quelconque des revendications précédentes,
**caractérisé par**
une composition de la matière de revêtement exempte de solvants ou exempte d'ajout de tensioactifs ou aussi bien exempte de solvants qu'exempte d'ajout de tensioactifs.

13. Procédé de revêtement thermofusible selon l'une quelconque des revendications précédentes,
**caractérisé**
**en ce que** la matière dispersée comporte au moins un principe actif pharmaceutique.

14. Procédé de revêtement thermofusible selon l'une quelconque des revendications précédentes,
**caractérisé**
**en ce que** la matière dispersée est constitués de cristaux d'un ou de plusieurs principe(s) actif(s) pharmaceutique(s).

15. Procédé de revêtement thermofusible selon l'une quelconque des revendications 13 ou 14,
**caractérisé**
**en ce qu'**au moins l'un des principes actifs pharmaceutiques est thermolabile et après l'enrobage et refroidissement consécutif à température ambiante fait preuve de plus de 98 % de son activité efficace initiale.
